**Europäisches Patentamt**

**European Patent Office**

**Office européen des brevets**

(19)

(11) Veröffentlichungsnummer: **0 200 151 B1**

# EUROPÄISCHE PATENTSCHRIFT

(12)

(45) Veröffentlichungstag der Patentschrift: **19.06.91**

(51) Int. Cl.⁵: **C07D 251/28**

(21) Anmeldenummer: **86105613.3**

(22) Anmeldetag: **23.04.86**

Die Akte enthält technische Angaben, die nach dem Eingang der Anmeldung eingereicht wurden und die nicht in dieser Patentschrift enthalten sind.

(54) **Verfahren zur Gewinnung von festem Cyanurchlorid.**

(30) Priorität: **24.04.85 DE 3514840**

(43) Veröffentlichungstag der Anmeldung:
**10.12.86 Patentblatt 86/45**

(45) Bekanntmachung des Hinweises auf die Patenterteilung:
**19.06.91 Patentblatt 91/25**

(84) Benannte Vertragsstaaten:
**BE DE GB IT NL**

(56) Entgegenhaltungen:
**EP-A- 0 137 505**
**EP-A- 0 158 362**
**DE-A- 1 544 129**

(73) Patentinhaber: **SKW Trostberg Aktiengesellschaft**
**Dr.-Albert-Frank-Strasse 32 Postfach 1150/1160**
**W-8223 Trostberg(DE)**

(72) Erfinder: **Klima, Hubertus**
**Fiedlerstrasse 3**
**W-8221 Tacherting(DE)**

(74) Vertreter: **Weickmann, Heinrich, Dipl.-Ing. et al**
**Patentanwälte Dipl.-Ing. H.Weickmann Dipl.-Phys.Dr. K.Fincke Dipl.-Ing. F.A.Weickmann Dipl.-Chem. B. Huber Dr.-Ing. H. Liska Dipl.-Phys.Dr. J. Prechtel Postfach 860820**
**W-8000 München 86(DE)**

## Beschreibung

Gegenstand der vorliegenden Erfindung ist ein Verfahren zur Gewinnung von festem Cyanurchlorid aus dem bei der Trimerisierung von Chlorcyan anfallenden Cyanurchloriddampf.

Cyanurchlorid besitzt als technisches Zwischenprodukt für die Herstellung von Farbstoffen, Pflanzenschutzmitteln, Pharmazeutika sowie Textil- und Kautschukhilfsmitteln erhebliche Bedeutung und fällt nach der katalytischen Trimerisierung von Chlorcyan als Gas zusammen mit unumgesetzten Chlorcyan und Chlor an. Dieses Gasgemisch wird üblicherweise in Abscheidekammern geleitet und das Cyanurchlorid an den gekühlten Wänden abgeschieden. Nachteilig bei dieser Art der Desublimation ist der Umstand, daß sich das Cyanurchlorid in Form grober Kristalle an den Wänden und Austragsvorrichtungen festsetzt und somit den Wärmeübergang negativ beeinflußt. Das regelmäßige Abklopfen der Anbackungen von den Wänden führt lediglich zu einer kurzzeitigen Verbesserung des Wärmeübergangs. Darüber hinaus ist diese Methode wegen der zunehmenden Beschädigung des Abscheiders und Lärmbelästigung keineswegs befriedigend, ganz abgesehen von der schlechten Qualität des auf diese Weise erhaltenen Produkts.

Gemäß der DE-AS 12 66 308 hat man versucht, dieses Problem dadurch zu lösen, daß man das Cyanurchlorid zusammen mit einer leicht verdampfenden Kühlflüssigkeit wie z.B. Methylenchlorid oder Chloroform versprühte. Man erhält auf diese Weise zwar ein feinverteiltes Cyanurchlorid, doch ist die Rückgewinnung der Kühlflüssigkeit technisch sehr aufwendig. Darüber hinaus kommt es sehr leicht zu Verstopfungen der Düse.

Anstelle der direkten Abscheidung des Cyanurchloriddampfes wurde z. B. gemäß der DE-PS 25 37 673 und DE-PS 23 32 636 vorgeschlagen, das im Reaktionsgas enthaltene Cyanurchlorid vor der Verfestigung zu verflüssigen und anschließend zu versprühen, wobei die Abführung der Desublimationswärme geringere Probleme aufwirft und Chlor sowie Chlorcyan vor der Abscheidung entfernt werden können. Dieses zweistufige Verfahren ist technisch ziemlich aufwendig. Diesen Nachteil weisen auch die Verfahren gemäß der deutschen Auslegeschriften 28 43 381 und 28 ,43 382 auf, bei denen man das nach der Trimerisierung von Cyanurchlorid anfallende Reaktionsgemisch in eine Apparatekombination, bestehend aus Abtriebskolonne und Kondensator, einleitet und durch Temperaturregelung am Austritt des Kondensators das Cyanurchlorid in der Kolonne teilweise kondensiert, während der gasförmige Anteil, der am Kolonnenkopf austritt, in den üblichen Abscheidekammern desublimiert wird. Dieses Verfahren verursacht jedoch hohe Betriebs- und Investitionskosten.

Nach DE-A-1544129 erfolgt die Abscheidung von festen Teilchen aus Gasen nicht an festen Wänden, sondern in der Berührungszone zweier gasförmiger Medien, wobei sich diese in Form von koaxialen gegenläufigen Drehströmen in einem Hohlraum (Rohr) bewegen.

Der vorliegenden Erfindung lag daher die Aufgabe zugrunde, ein Verfahren zur Gewinnung von festem Cyanurchlorid aus dem bei der Trimerisierung von Chlorcyan anfallenden Cyanurchloriddampf zu entwickeln, das die genannten Nachteile des Standes der Technik nicht aufweist, und das es ermöglicht, ein feinkörniges Cyanurchlorid mit einem engen Kornspektrum mit technisch einfachen Mitteln herzustellen.

Diese Aufgabe wurde erfindungsgemäß durch ein Verfahren zur Gewinnung von festem Cyanurchlorid aus dem bei der Trimerisierung von Chlorcyan anfallenden Cyanurchloriddampf gemäß Patentanspruch 1 gelöst.

Zweckmäßige Ausgestaltungen dieses Verfahrens sind Gegenstand der Ansprüche 2 bis 6.

Es hat sich nämlich überraschenderweise gezeigt, daß sich mit Hilfe des erfindungsgemäßen Verfahrens ein sehr reines Cyanurchlorid, mit einem wesentlich höheren Durchsatz, als es mit bekannten Verfahren möglich ist, gewinnen läßt. Das so erhaltene Cyanurchlorid ist feinteilig und hat eine günstige Verteilung der Korngrößen.

Gemäß der vorliegenden Erfindung wird das bei der Trimerisierung von Chlorcyan anfallende Reaktionsgemisch so in den unteren Teil einer Abscheidekammer eingeleitet, daß es sich im Zentrum vieler einzelner Inertgasströme befindet, die sich von unten nach oben bewegen. Auf diese Weise wird verhindert, daß der Cyanurchloriddampf mit den Wänden des Abscheiders in Berührung kommt und daß sich hierbei grobe Kristalle oder Agglomerate bilden. Die Aufspaltung des Inertgasstromes in einzelne Teilströme kann mit den üblichen Düsen erfolgen, die konzentrisch um die Cyanurchloridzuleitung angeordnet sind.

In einer bevorzugten Ausführungsform werden der Cyanurchloriddampf und die Inertgasströme mit Hilfe eines Treibstrahls umgewälzt.

In einer besonders bevorzugten Ausführungsform erfolgt die Zufuhr der Inertgasströme mit Hilfe einer Ringdüse, die aus einem Rohrring mit einer entsprechenden Anzahl von Löchern besteht und die als Jetring bezeichnet wird. Durch diesen Jetring treten die Inertgasteilströme mit hoher Geschwindigkeit in die Abscheidekammer ein. Durch die hohe Geschwindigkeit der Inertgasströme entsteht im Zentrum des Jetrings ein Unterdruck, der eine Gaszirkulation bewirkt. Das aufsteigende Gas, welches die kondensierten Cyanurchloridteilchen mitreißt, Wird nämlich am Kopf des Abscheiders umgelenkt und anschließend an den Wänden nach unten gesogen und dort gekühlt. In der Höhe der Ringdüse erfahren die Inertgasströme erneut einen Richtungswechsel und werden wieder nach oben gelenkt. An dieser Stelle erfolgt die Trennung der Cyanurchloridpartikel vom Inertgas. Durch die dort wirksame Zentrifugal- bzw. Schwerkraft werden die größeren Partikel auf den Boden des Abscheiders geschleudert und können dort problemlos mit Hilfe der üblichen Austragsvorrichtungen ausgeschleust werden. Die kleineren Cyanurchlorid-Teilchen werden noch einmal angesaugt und werden zusammen mit der Zirkulationsströmung nach oben befördert. Dabei dienen diese Feinpartikel als Kristallisationskeime, welche die Kondensation des neu eintretenden Cyanurchloriddampfes beschleunigen. Auf diese Weise entsteht ein sehr enges Korngrößenspektrum, welches sowohl durch die Temperatur als auch durch den Massenstrom des Inertgases exakt gesteuert werden kann.

Die Inertgasströme werden mit sehr hoher Geschwindigkeit von mehr als 80m/s, in die Abscheidekammer eingeleitet. Bevorzugt werden Inertgasströme mit Schallgeschwindigkeit in die Abscheidekammer eingeleitet. Durch diese hohe Geschwindigkeit wird das Cyanurchlorid und das Inertgas als Treibstrahl in der Abscheidekammer umgewälzt, wobei eine rasche Durchmischung des Cyanurchloriddampfes mit dem Inertgas erfolgt.

Die Inertgasströme haben zweckmäßigerweise eine Temperatur von 0°C bis +40°C. Im erfindungsgemäßen Temperaturbereich ist der Abschreckeffekt in jedem Fall ausreichend, so daß die Cyanurchloridteilchen in der gewünschten feinteiligen Form anfallen.

Das Massenstrom-Verhältnis von Cyanurchloriddampf zu Inertgas ist nicht kritisch und kann in weiten Grenzen variiert werden. Bevorzugt wird ein Massenstromverhältnis von Cyanurchloriddampf zu Inertgas von 0,5 bis 5, insbesondere 1,5 bis 3, eingestellt.

Als Inertgas können alle Gase eingesetzt werden, die mit dem Cyanurchloriddampf bei den entsprechenden Temperaturen keine Reaktion eingehen. Aus wirtschaftlichen Gründen werden bevorzugt trockene Luft oder Stickstoff eingesetzt.

Das überschüssige, erwärmte Inertgas, welches noch geringe Anteile an Feinstpartikeln sowie Verunreinigungen an Chlorcyan und Chlor enthält, wird bevorzugt im unteren Teil des Abscheiders seitlich abgezogen. Nach Abkühlung und Entfernung der Verunreinigungen nach bekannten Methoden kann das Inertgas dem Abscheider wieder zugeführt werden.

Mit Hilfe des erfindungsgemäßgne Verfahrens lassen sich hohe Abscheidegeschwindigkeiten bzw. Kondensationsdurchsätze erzielen, ohne daß es zu nennenswerten Anbackungen kommt. Gleichzeitig kann ein sehr feinteiliges Cyanurchlorid erhalten werden, dessen Schüttgewicht unter 800 kg/m³ liegt.

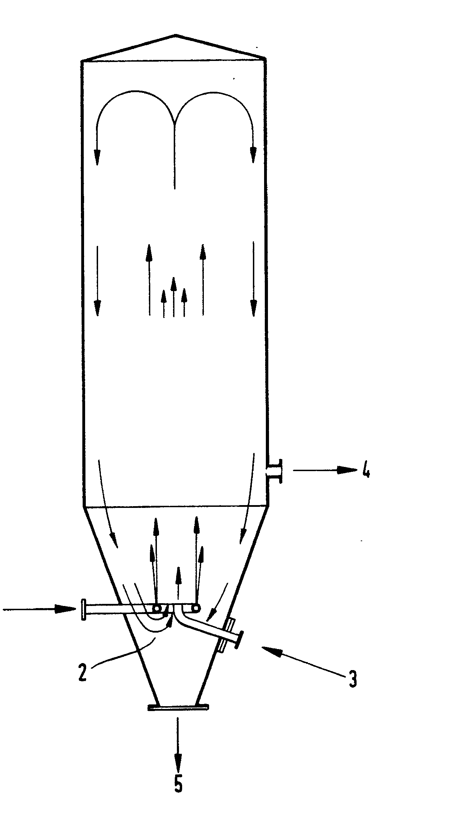

Figur 1 zeigt eine bevorzugte Ausführungsform des erfindungsgemäßen Verfahrens.

Gemäß Figur 1 tritt das gekühlte Inertgas durch Leitung 1 in die Abscheidekammer 2 ein und wird mit Hilfe des Jetrings in viele Teilströme hoher Geschindigkeit zerlegt. In das Zentrum dieser Teilströme wird über Leitung 3 der Cyanurchloriddampf dem Abscheider zugeführt. Nach der Abscheidung des Cyanurchlorids wird das überschüssige, erwärmte Inertgas über Leitung 4 abgezogen und das abgeschiedene Cyanurchlorid am Boden des Abscheiders über Leitung 5 ausgeschleust.

Das nachfolgende Beispiel erläutert die Erfindung, ohne sie zu beschränken:

Beispiel:

Gemäß Figur 1 wurde ein Versuch durchgeführt, bei dem Cyanurchlorid unter folgenden Bedingungen abgeschieden wurde:
Massenstrom an Cyanurchlorid: 100 Gew.-teile/h
Massenstrom an Inertgas: 50 Gew.-teile /h
Temperatur des eintretenden Inertgases: 20°C Geschwindigkeit des eintretenden Inertgases: 330m/sec.

Es wurde Cyanurchlorid mit einem Schüttgewicht von 760 kg/m³ erhalten.

## Ansprüche

1. Verfahren zur Gewinnung von festem Cyanurchlorid aus dem bei der Trimerisierung von Chlorcyan anfallenden Cyanurchloriddampf, dadurch gekennzeichnet, daß man den Cyanurchloriddampf im unteren teil des Abscheiders in das Zentrum vieler einzelner, kalter, ringförmig angeordneter Inertgasströme, die eine Geschwindigkeit von mehr als 80 m/s und eine Temperatur von -80°C bis +100°C besitzen, einleitet und das am Boden des Abscheiders anfallende feste Cyanurchlorid austrägt, während das erwärmte Inertgas seitlich abgezogen wird.

2. Verfahren nach Anspruch 1, dadurch gekennzeichnet, daß man das Cyanurchlorid und das Inertgas mit Hilfe eines Treibstrahls umwälzt.

3. Verfahren nach einem der Ansprüche 1 bis 2, dadurch gekennzeichnet, daß man die Inertgasströme durch eine Ringdüse in den Abscheider einleitet.

4. Verfahren nach einem der Ansprüche 1 bis 3, dadurch gekennzeichnet, daß man die Inertgasströme mit Schallgeschwindigkeit einleitet.

5. Verfahren nach einem der Ansprüche 1 bis 4, dadurch gekennzeichnet, daß man Inertgasströme mit einer Temperatur von 0˚C bis +40˚C einleitet.

6. Verfahren nach einem der Ansprüche 1 bis 5, dadurch gekennzeichnet, daß man das Massenstromverhältnis von Cyanurchloriddampf zu Inertgas auf 0.5 bis 5, vorzugsweise 1,5 bis 3 einstellt.

**Claims**

1. Process for the obtaining of solid cyanuric chloride from the cyanuric chloride vapour obtained by the trimerisation of cyanogen chloride, characterised in that one introduces the cyanuric chloride vapour into the lower part of separator in the centre of many individual, cold, annularly disposed inert gas streams, which possess a speed of more than 80 m./s. and a temperature of -80˚C. to +100˚C., and removes the solid cyanuric chloride depositing on the bottom of the separator, whereas the heated inert gas is removed laterally.

2. Process according to claim 1, characterised in that one circulates the cyanuric chloride and the inert gas with the help of a propulsion jet.

3. Process according to one of claims 1 or 2, characterised in that one introduces the inert gas streams through a ring nozzle into the separator.

4. Process according to one of claims 1 to 3, characterised in that one introduces the inert gas streams at sonic speed.

5. Process according to one of claims 1 to 4, characterised in that one introduces the inert gas streams with a temperature of 0˚C. to +40˚C.

6. Process according to one of claims 1 to 5, characterised in that one adjusts the mass-stream ratio of cyanuric chloride vapour to inert gas to 0.5 to 5, preferably 1.5 to 3.

**Revendications**

1. Procédé de récupération du chlorure de cyanuryle solide provenant des vapeurs de chlorure de cyanuryle résultant de la trimérisation du chlorure de cyanogène, caractérisé en ce que l'on introduit les vapeurs de chlorure de cyanuryle dans la partie inférieure du séparateur, au centre de nombreux courants séparés et de forme annulaire de gaz inerte froid ayant une vitesse de plus de 80 m/sec. et une température de -80˚C à +100˚C et que l'on évacue le chlorure de cyanuryle solide déposé sur le fond du séparateur tandis que le gaz inerte chauffé est aspiré latéralement.

2. Procédé selon la revendication 1, caractérisé en ce que l'on mélange le chlorure de cyanuryle et le gaz inerte à l'aide d'un jet propulseur.

3. Procédé selon l'une des revendications 1 et 2, caractérisé en ce que l'on introduit les courants de gaz inerte dans le séparateur par une tuyère annulaire.

4. Procédé selon l'une des revendications 1 à 3, caractérisé en ce que l'on introduit les courants de gaz inerte à la vitesse du son.

5. Procédé selon l'une des revendications 1 à 4, caractérisé en ce que l'on introduit les courants de gaz inerte à une température de 0˚C à +40˚C.

6. Procédé selon l'une des revendications 1 à 5, caractérisé en ce que l'on règle le rapport des débits massiques des vapeurs de chlorure de cyanuryle et de gaz inerte dans l'intervalle allant de 0,5 à 5 et, de préférence, allant de 1,5 à 3.